# EUROPEAN PATENT APPLICATION

(11) **EP 3 939 956 A1**
(43) Date of publication of application: **19.01.2022**
(21) Application number: 20186577.1
(22) Date of filing: 17.07.2020
(51) Int. Cl.: C07C 31/20, C07C 229/12, C07C 227/18, C09K 3/00, C11D 1/00

(54) **NEW DIOLS AND NEW QUATERNARY AMMONIUM COMPOUNDS**

(71) Applicant: Rhodia Operations, 93300 Aubervilliers (FR)
(72) Inventor: BACK, Oliver, 69008 Lyon (FR); MASTRIOIANNI, Sergio, 69006 Lyon (FR)
(74) Representative: Ridray, Annabelle

(57) **Abstract**

The invention concerns new diols of formula (III) 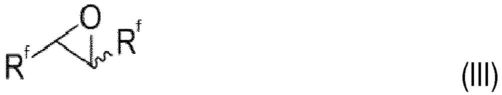 wherein R^{f}, which may be the same or different at each occurrence, represents a C₁₀-C₂₇ aliphatic group, their synthesis and their use for the manufacture of new quaternary ammonium compounds with outstanding surfactant properties and improved biodegradability.

## Description

The present invention relates to new quaternary ammonium compounds and their use as surfactants. It relates also to new diols useful for the manufacture of such quaternary ammonium compounds.

Fatty quaternary ammonium compounds which have surfactant properties and can be used in respective applications have been described in the literature and are available commercially in a variety of different types from various suppliers.

WO 97/08284 discloses compositions comprising Guerbet alcohol betaine esters which are represented by the general formula 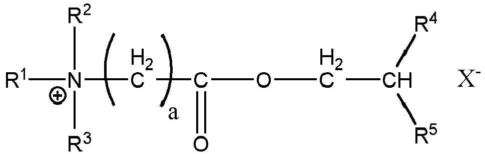 in which R¹ to R³ are independently selected from C₁ to C₄ alkyl groups or C₂-C₄ alkenyl groups, a is from 1 to 4 and R₄ and R₅ are independently selected from C₁₂ to C₂₂ alkyl or alkenyl groups, the sum of chain lenghts of R₄ and R₅ preferably being at least 30. Since the compounds are derived form Guerbet alcohols, the number of carbon atoms in groups R₄ and R₅ differs always by 2.

EP 721 936 is related to liquid quaternary ammonium compounds of the general formula 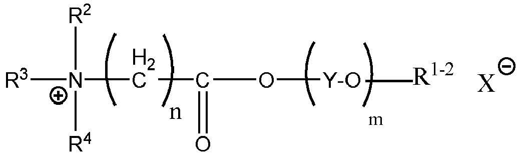 wherein R¹⁻² is a linear or branched C₃₆-C₄₄ alkyl or alkenyl group, R₂ to R₄ are C₁-C₅ alkyl or hydroxyalkyl groups, Y is a linear or branched C₂-C₄ alkylene group, m is a number of 0 to 20 and n is an integer of 1 to 6. Preferred compounds of EP 721 936 are, as in WO 97/08284, derived from Guerbet alcohols and are represented by the formula

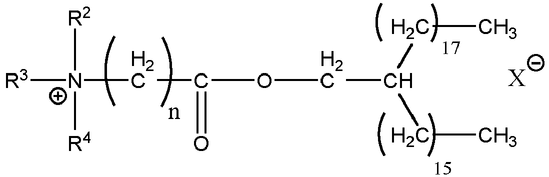

DE 3402146 relates to quaternary ammonium compounds. As in WO 97/08284 and EP 721 936, the compounds comprise two long chain substituents which are esters of Guerbet acids.

While fatty quaternary ammonium compounds are widely used as surfactants, there is still a need for compounds of this type having a good combination of surfactant properties on one hand and biodegradability on the other hand. Biodegradability has become more and more important in the recent past due to the desire of customers to have more environmentally friendly products. The improvement in biodegradability should not negatively affect the surfactant properties.

It was thus an object of the present invention to provide new quaternary ammonium compounds with good surfactant properties and a good biodegradability.

This object is achieved with the compounds of formula (I). Preferred embodiments of the present invention are also detailed hereinafter.

The novel ionic compounds in accordance with the present invention have the general formula (I) 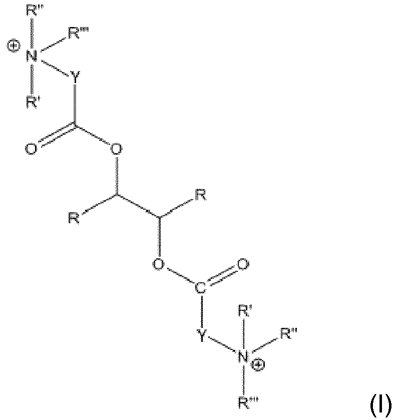 wherein
R, which may be the same or different at each occurrence, is a C₅-C₂₇ aliphatic group, preferably a C₆ to C₂₄ aliphatic group,
Y, which may be the same or different at each occurrence, is a divalent C₁-C₆ aliphatic radical,
R', R" and R'", which may be the same or different at each occurrence, are, independently from each other, hydrogen or a C₁ to C₄ alkyl group,

The aliphatic groups R may be free of any double bond and of any triple bond. Alternatively, the aliphatic groups R may comprise at least one -C=C- double bond and/or at least one -C≡C- triple bond.

The aliphatic groups R are advantageously chosen from alkyl groups, alkenyl groups, alkanedienyl groups, alkanetrienyl groups and alkynyl groups.

The aliphatic groups R may be linear or branched.

Preferably, the aliphatic groups R are independently chosen from alkyl and alkenyl groups.

More preferably, the aliphatic groups R are independently chosen from alkyl and alkenyl groups, generally from C₆-C₂₄ alkyl and C₆-C₂₄ alkenyl groups, very often from C₆-C₂₁ alkyl and C₆-C₂₁ alkenyl groups and often from (i) C₆-C₁₉ alkyl and C₆-C₁₉ alkenyl groups or from (ii) C₆-C₁₇ alkyl and C₆-C₁₇ alkenyl groups. More preferably, R represent an alkyl group, generally a C₆-C₂₄ alkyl group, very often a C₆-C₂₁ alkyl group, often a C₆-C₁₉ alkyl group or a C₆-C₁₇ alkyl group. Aliphatic groups, in particular alkyl groups, with 10 to 20, preferably with 11 to 17 or with 10 to 17 carbon atoms have been found advantageous in certain cases.

Acyclic aliphatic groups, more preferably linear aliphatic groups, still more preferably linear alkyl groups may be mentioned as preferred examples of substituents R.

The number of carbon atoms of R can be even or odd and each group R can have the same number of carbon atoms or the number of carbon atoms of different groups R may be different.

R', R" and R'", which may be the same or different, are preferably hydrogen or a C₁ to C₄ alkyl group, preferably methyl or ethyl, more preferably methyl. Preferably at least one, more preferably at least two, more preferably all three of R', R" and R'" are a C₁ to C₄ alkyl group, preferably methyl or ethyl, most preferably methyl.

Y is preferably an acyclic divalent aliphatic group, more preferably a linear divalent aliphatic group, still more preferably a linear alkanediyl (alkylene) group and preferably has 1 to 6, even more preferably 1 to 4 carbon atoms. In compounds where n' is 1, aliphatic group Y preferably has at least two carbon atoms, in particular 2 to 6 carbon atoms.

The compounds of formula (II) 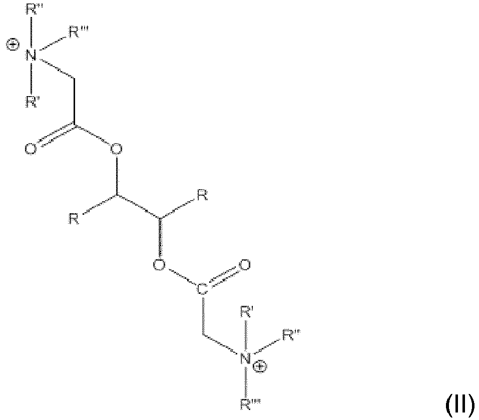 wherein R, R', R", R'" and Y in formula (II) have the meaning as defined for formula (I) as described hereinbefore represent particularly preferred compounds in accordance with the present invention.

The compounds in accordance with the present invention can be obtained by a variety of methods. Preferred processes for the manufacture of the compounds of the present invention include the reaction of an internal ketone of formula R-C(=O)-R, which internal ketone may preferably be obtained by decarboxylative ketonization of a fatty acid, a fatty acid derivative or a mixture thereof. A suitable process for the manufacture of internal ketones following this route is diclosed in US 2018/0093936 to which reference is made for further details.

The synthesis of various compounds of the present invention using internal ketones obtainable as indicated above as starting materials is now described. The process variants described hereinafter show the synthesis of specific compounds and the skilled person will modify the reactants and reaction conditions based on his professional knowledge and taking into account the specific target product of the respective synthesis to manufacture other compounds in accordance with the present invention.

The compounds of formula (I) can preferably be obtained by two processes. The first process starts with a *Piria ketonization* followed by hydrogenation, dehydration, epoxydation (to obtain an epoxide), hydration (to obtain a diol) and esterification (to obtain a certain diester). This is a multi-step process plugged on Piria technology. It has the advantage of being salt-free and relying on chemical transformations which can be easily performed.

As an alternative to the above sequence of reactions, it is possible to bypass the hydration step (i.e. the formation of the diol) by converting straightforward the epoxide into the diester provided an appropriate esterification agent is used, as will be detailed later on.

The esterification step may be followed by an amine condensation step (as the final step) to convert the diester into a compound complying with formula (I).

Finally, starting from the diol, it also possible to obtain in one reaction step a compound which complies with formula (I), i.e. to achieve in one step said esterification and amine condensation, provided another appropriate esterification is used, as will be detailed later on.

### First process for synthesis of compounds represented by formula (I)

### Piria Ketonization

The basic reaction in the first step is:

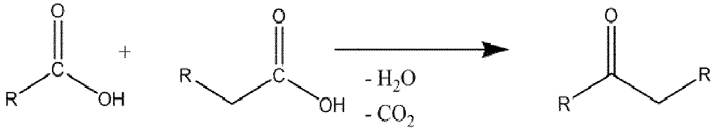

This reaction has been thoroughly described in US patent 10035746, WO 2018/087179 and WO 2018/033607 to which reference is made for further details.

### Hydrogenation

The internal ketone is then subjected to hydrogenation which can be carried out under standard conditions known to the skilled person for hydrogenation reactions:

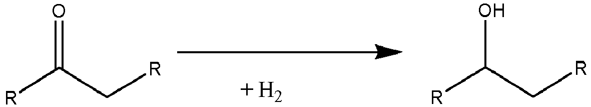

The hydrogenation reaction is conducted by contacting the internal ketone with hydrogen in an autoclave reactor at a temperature ranging from 15°C to 300°C and at a hydrogen pressure ranging from 1 bar to 100 bars. The reaction can be conducted in the presence of an optional solvent but the use of such solvent is not mandatory and the reaction can also be conducted without any added solvent. As examples of suitable solvents one can mention: methanol, ethanol, isopropanol, butanol, THF, methyl-THF, hydrocarbons, water or mixtures thereof. A suitable catalyst based on a transition metal should be employed for this reaction. As examples of suitable catalysts, one can mention heterogeneous transition metal based catalysts such as for example supported dispersed transition metal based catalysts or homogeneous organometallic complexes of transition metals. Examples of suitable transition metals are: Ni, Cu, Co, Fe, Pd, Rh, Ru, Pt, Ir. As examples of suitable catalysts one can mention Pd/C, Ru/C, Pd/Al₂O₃, Pt/C, Pt/Al₂O₃, Raney Nickel, Raney Cobalt etc. At the end of the reaction, the desired alcohol can be recovered after appropriate work-up. The skilled person is aware of representative techniques so no further details need to be given here. Details of this process step can e.g. be found in US patent 10035746 to which reference is made here.

The skilled person will select suitable reaction conditions based on his professional experience and taking into account the specific target compound to be synthesized. Accordingly, no further details need to be given here.

### Dehydration

In the next step, the alcohol thus obtained is subjected to dehydration to obtain an internal olefin. This reaction can also be carried out under standard conditions known to the skilled person for respective dehydration reactions (e.g. US patent 10035746, example 4) so that no further details need to be given here:

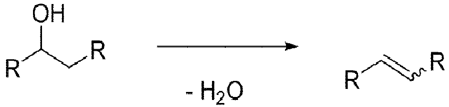

The dehydration reaction is conducted by heating the secondary alcohol in a reaction zone in the presence of a suitable catalyst at a temperature ranging between 100°C and 400°C. The reaction can be conducted in the presence of an optional solvent but the use of such solvent is not mandatory and the reaction can also be conducted without any added solvent. As examples of solvents one can mention: hydrocarbons, toluene, xylene or their mixture. A catalyst must be employed for this reaction. Suitable examples of catalysts are acidic (Lewis or Bronsted) catalysts either heterogeneous solid acid catalysts or homogeneous catalysts. As examples of heterogeneous catalysts one can mention alumina (Al₂O₃), silica (SiO₂), aluminosilicates (Al₂O₃-SiO₂) such as zeolites, phosphoric acid supported on silica or alumina, acidic resins such as Amberlite^{®} etc. Homogeneous catalysts can also be employed and one can mention the following suitable acids: H₂SO₄, HCI, trifluoromethanesulfonic acid, *para-*toluenesulfonic acid, AlCl₃, FeCl₃ etc. Water that is generated during the reaction can be distilled out from the reaction medium in the course of the reaction. At the end of the reaction, the desired olefin can be recovered after appropriate work-up. The skilled person is aware of representative techniques and same are e.g. described in US patent 10035746 so that no further details need to be given here.

### Epoxidation

This internal olefin can thereafter be oxidized to the respective epoxide wherein the double bond is substituted by an epoxide group in accordance with the following scheme (where the reactants are just exemplary for respective groups of compounds serving the respective function): 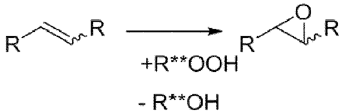 wherein R** can be hydrogen or a hydrocarbon group that can be substituted and/or interrupted by a heteroatom or heteroatom containing group, or R** can be an acyl group of general formula R***-C(=O)-wherein R*** can have the same meaning as R**.

The epoxidation reaction is advantageously conducted by contacting the internal olefin with an appropriate oxidizing agent in a reaction zone at a temperature ranging usually from 15°C to 250°C.

As suitable oxidizing agents one can mention peroxide compounds such as hydrogen peroxide (H₂O₂) that can be employed in the form of an aqueous solution, organic peroxides such as peracids of general formula R****-CO₃H (for example *meta*-chloroperoxybenzoic acid, peracetic acid, etc.), hydrocarbyl (e.g. alkyl) hydroperoxides of general formula R****'-O₂H (for example cyclohexyl hydroperoxide, cumene hydroperoxide, *tert*-butyl hydroperoxide) where R**** in the peracid or R****' in the hydrocarbyl (e.g. alkyl) hydroperoxide is a hydrocarbon group (e.g. an alkyl group) that can be substituted and/or interrupted by a heteroatom or heteroatom-containing group.

The reaction can be conducted in the presence of an optional solvent but the use of such solvent is not mandatory and the reaction can also be conducted without any added solvent. As example of suitable solvents one can mention: CHCl₃, CH₂Cl₂, *tert*-butanol or their mixtures.

When H₂O₂ is used as the oxidizing agent, the presence of an organic carboxylic acid during the reaction can be beneficial as it will generate in-situ a peracid compound by reaction with H₂O₂. As examples of suitable carboxylic acids one can mention: formic acid, acetic acid, propionic acid, butanoic acid, benzoic acid etc.

A catalyst can also be used to promote the reaction. Suitable catalysts are Lewis or Bronsted acids and one can mention for example: perchloric acid (HClO₄), trifluoromethanesulfonic acid, heterogeneous titanium silicalite (TiO₂-SiO₂), heterogeneous acidic resins such as Amberlite^{®} resins, homogeneous organometallic complexes of manganese, titanium, vanadium, rhenium, tungsten, polyoxometellates etc.

At the end of the reaction, the desired epoxide can be recovered after appropriate work-up and the skilled person is aware of representative techniques so that no further details need to be given here.

The epoxide can be directly engaged in next step without further purification. This next step can be a hydration step (so as to form a diol, to be the followed by an esterification step) or a direct esterification step.

### Epoxide hydration (diol formation)

The epoxide can thereafter be hydrated to the respective diol in accordance with the following scheme:

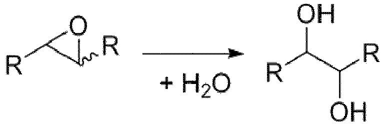

The ring opening reaction can be performed by contacting the epoxide with water, generally in the presence of a suitable catalyst and at a temperature ranging generally from 15°C to 150°C. As examples of catalysts one can mention Bronsted or Lewis acid catalysts such as: H₂SO₄, HCl, perchloric acid (HClO₄), trifluoromethanesulfonic acid, *para-*toluenesulfonic acid, heterogeneous acidic resins such as Amberlite^{®} resins etc.

The reaction can be conducted in the presence of an optional solvent to facilitate reagent contact and one can mention: Me-THF, THF, DMSO, *tert-*butanol, methanol, ethanol, isopropanol, acetonitrile, or their mixture. The reaction can also be conducted without any added solvent.

At the end of the reaction, the desired diol can be recovered after appropriate work-up and the skilled person is aware of representative techniques so that no further details need to be given here.

Amongst the diols of formula 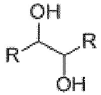 wherein R, which may be the same or different at each occurrence, represents in general a C₅-C₂₇ aliphatic group, those wherein R has at least 10 carbon atoms, especially those wherein R has from 10 to 20 carbon atoms, have likewise been found particularly advantageous for the preparation of compounds of formula (I) useful as surfactants demonstrating an outstanding balance of properties. Yet, such diols, which can be represented by formula (III) 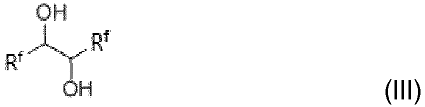 wherein R^{f}, which may be the same or different at each occurrence, represents a C₁₀-C₂₇ aliphatic group, have been rarely disclosed in the literature and such rare disclosures were totally unrelated to the technical field of the present invention, i.e. the synthesis of surfactants.

The *website of PubChem* discloses several alkanediols complying with formula (III). Except a few alkanediols which will be cited and commented thereafter, the alkanediols of PubChem appear to be pure theoretical constructions their properties of which were estimated by predictive models. As such alkanediols, it can be listed the following alkanediols: docosane-11,12-diol, tricosane-11,12-diol, tetracosane-12,13-diol, pentacosane-12,13-diol, hexacosane-12,13-diol, heptacosane-11,12-diol, octacosane-12,13-diol, octacosane-14,15-diol, nonacosane-14,15-diol, triacontane-15,16-diol, hentriacontane-15,16-diol, dotriacontane-14,15-diol, dotriacontane-15,16-diol, dotriacontane-16,17-diol, tetratriacontane-16,17-diol, tetratriacontane-17,18-diol, pentatriacontane-17,18-diol, hexatriacontane-15,16-diol, heptatriacontane-12,13-diol, octatriacontane-18,19-diol, octatriacontane-19,20-diol, nonatriacontane-19,20-diol, tetracontane-18,19-diol, tetracontane-19,20-diol, dotetracontane-20,21-diol and tetratetracontane-22,23-diol ("Pubchem" list). Beyond the fact that the disclosure of the diols forming part of "Pubchem" list has obviously no connection with the present invention and appears as such to be accidental, it is just not enabling because Pubchem does not provide any indication on how to make these diols.

Tornabene et al., in Lipids, Vol. 6, No. 3, pp. 190-195 (1971), proceeded with the characterization of branched monounsaturated hydrocarbons of Sarcina lutea and Sarcina flava. Within the frame of this characterization, olefinic hydrocarbons were converted into trimethylsilyl ether (TMSE) derivatives ; obtaining such TMSE derivatives went through the formation of diol intermediates by oxidizing the olefinic hydrocarbons with osmium tetroxide. These characterization efforts resulted in the identification of some C₂₃-C₃₀ branched olefins: branched 11-tricosene, branched 11-tetracosene, branched 12-tetracosene, branched 12-pentacosene, branched 12-hexacosene, branched 13-hexacosene, branched 12-heptacosene, branched 13-heptacosene, branched 13-octacosene, branched 14-octacosene, branched 14-nonacosene and branched 15-triacontene. While this should not be construed as an admission from the Applicant, it could perhaps be presumed that diol intermediates corresponding to the previously cited olefins were obtained when forming the TMSE derivatives useful for the characterization. Besides, two GC peaks (named peaks "a" and "b" in Table 1) were tentatively (emphasis added) attributed to linear 13-heptacosene and linear 14-nonacosene. Tornabene's disclosure has no connection with the present invention and, should it be considered relevant for the assessment of the novelty of the invention, it would be held accidental.

Subramanian et al., in Tetrahedron, Vol. 42, No. 14, pp.3967-3972 (1986) describes a multi-step process for the synthesis of 13-heptacosene. Heptacosane-13,14-diol, which served as an intermediate in this synthesis, was obtained by firstly reducing the isopropylidene derivative of dimethyl-13,14-dihydroxyheptacosa-1,27-dioate with lithium aluminium hydride to give 1,13,14,27-tetrahydroxyheptacosane, then tosylating the acetonide of above tetrol, reducing with Zn-Nal and hydrolysing with an acid. This disclosure of diols complying with formula (III) has no connection with the present invention and appears as such to be accidental.

KR20060060776 (to Eung Ju Oh) describes the synthesis of poly[3,4-alkoxythiophenes] and poly[3,4-ethylenedioxythiophenes] useful in organic electronic applications. Docosane-11,12-diol is involved in a synthesis of such polymers. This other disclosure of a diol complying with formula (III) has obviously no connection with the present invention and appears also as such to be accidental.

It was thus another object of the present invention to provide new diols useful for the manufacture of quaternary ammonium compounds with good surfactant properties and a good biodegradability.

This other object is achieved with the diols which are described hereinafter under item 1:
- item 1: a diol of formula (III) 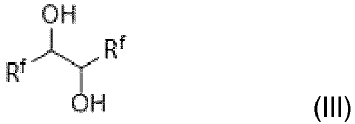
   wherein R^{f}, which may be the same or different at each occurrence, represents a C₁₀-C₂₇ aliphatic group,
   with the exception of docosane-11,12-diol and heptacosane-13,14-diol (without any particular limitation as to the isomeric forms, thus including linear heptacosane-13,14-diol and branched octacosane-13,14-diol).
   Preferred and/or particular embodiments related to the invented diols are set hereinafter:
- item 2: the diol as described in item 1 with the further exception of branched tricosane-11,12-diol, branched tetracosane-11,12-diol, branched tetracosane-12,13-diol, branched pentacosane-12,13-diol, branched hexacosane-12,13-diol, branched hexacosane-13,14-diol, branched heptacosane-12,13-diol, branched octacosane-13,14-diol, branched octacosane-14,15-diol, branched nonacosane-14,15-diol and branched triacontane-15,16-diol.
- item 3: the diol as described in item 2 with the further exception of linear nonacosane-14,15-diol.
- item 4: the diol as described in item 1 or 2 or 3 with the further exception of the alkanediols of "Pubchem" list as above detailed ;
- item 5: the diol as described in any one of items 1 to 4 wherein R^{f} (i.e. each R^{f}) is free of any double bond and of any triple bond ;
- item 6: the diol as described in any one of items 1 to 4 wherein R^{f} is an alkyl group ;
- item 7: the diol as described in any one of items 1 to 6 wherein R^{f} is linear;
- item 8: the diol as described in any one of items 1 to 7 wherein each R^{f} comprises at least 12 carbon atoms and at least one R^{f} comprises at least 13 carbon atoms ;
- item 9: the diol as described in item 8 wherein R^{f}(i.e. each R^{f}) comprises at least 14 carbon atoms ;
- item 10: the diol as described in any one of items 1 to 9 wherein R^{f} comprises at most 24 carbon atoms ;
- item 11: the diol as described in item 10 wherein R^{f} comprises at most 20 carbon atoms ;
- item 12: the diol as described in item 11 wherein R^{f} comprises at most 17 carbon atoms ;
- item 13: the diol as described in any one of items 1 to 7 wherein R^{f} comprises from 10 to 20 carbon atoms ;
- item 14: the diol as described in item 13 wherein R^{f} comprises from 11 to 17 carbon atoms ;
- item 15: the diol as described in any one of items 1 to 14 wherein the total number of carbon atoms of the two R^{f} groups is of at least 30 ;
- item 16: the diol as described in any one of items 1 to 7 wherein R^{f} is a linear alkyl group having from 14 to 17 carbon atoms ;
- item 17: the diol as described in any one of items 1 to 16 wherein one and only one R^{f} has an odd number of carbon atoms and one and only one R^{f} has an even number of carbon atoms possibly a C₃₃ diol, a C₄₁ diol, a C₄₅ diol, a C₄₇ diol, a C₄₉ diol, a C₅₁ diol, a C₅₃ diol or a C₅₅ diol ;
   this can happen when both R^{f} originate from a carboxylic acid having an even number of carbon atoms and can be advantageous from an economic standpoint because fatty carboxylic acids of natural origin -which have typically such an even number of carbon atoms- are broadly available ;
   this can also happen when both R^{f} originate from a carboxylic acid having an odd number of carbon atoms ;
- item 18: the diol as described in any one of items 1 to 16 either both R^{f} have an odd number of carbon atoms or both R^{f} have an even number of carbon atoms, possibly a C₄₄ diol, a C₄₆ diol, a C₄₈ diol, a C₅₀ diol, a C₅₂ diol, a C₅₄ diol or a C₅₆ diol ;
   this can happen when one and only one R^{f} originates from a carboxylic acid having an even number of carbon atoms and one and only one R^{f} originates from a carboxylic acid having an odd number of carbon atoms ; in practice, since carboxylic acids of different chain lengths have nonetheless generally close reactivities, a mixture of a first diol wherein both R^{f} have an even number of carbon atoms and of a second diol wherein both R^{f} have an odd number of carbon atoms is generally obtained ;
- item 19: the diol as described in any one of items 1 to 16 wherein one and only one R^{f} has an odd number of carbon atoms no while the other R^{f} has an even number of carbon atoms n_{E}, wherein n_{E} is equal to no-1; this can happen when the diol is obtained from one and only one carboxylic acid having an even number of carbon atoms and can also be advantageous from an economic standpoint for the same reason as above stated ;
- item 20: the diol as described in any one of items 1 to 19 but 18 wherein the number of carbon atoms of the two groups R^{f} is represented by couple (n₁, n₂), n₁ being the number of carbon atoms of the first R^{f} group and n₂ being the number of carbon atoms of the second R^{f} group, said couple (n₁, n₂) being chosen from the following couples:
   (10,11), (12,13), (14,15), (16,17), (10,13), (10,15), (10,17), (11,12), (11,14), (11,16), (12,15), (12,17), (13,14), (13,16), (14,17) and (15,16).
- item 21: the diol as described in item 20 wherein the couple (n₁, n₂) representing the number of carbon atoms of the two groups R^{f} is chosen from the following couples: (14,15), (16,17),(14,17) and (15,16) ;
- item 22: a pack of at least one kilogram comprising the diol as described in any one of items 1 to.21 ;
- item 23 : a diol mixture comprising a first diol characterized by R^{f} groups having respectively n₃ and (n₃-1) carbon atoms, a second diol characterized by R^{f} groups having respectively n₄ and (n₄-1) carbon atoms, a third diol characterized by R^{f} groups having respectively n₃ and (n₄-1) carbon atoms and a fourth diol characterized by R^{f} groups having respectively n₄ and (n₃-1) carbon atoms, wherein n₃ ≤ n₄ + 2 and wherein the first diol, the second diol, the third diol and the fourth diol are as described in any one of items 1 to 17 or in item 21 ;
- item 24: a method for obtaining the diol as described in any one of items 1 to 21 which comprises reacting an epoxide compound of formula 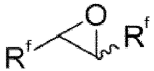
   wherein R^{f}, which may be the same or different at each occurrence, is as described here before for the diol,
   with water;
- item 25: the method as described in item 24 wherein the reaction is conducted in the presence of a Bronsted or Lewis catalyst;
- item 26: the method described in item 24 or 25 which comprises reacting an olefin of formula 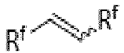
   wherein R^{f}, which may be the same or different at each occurrence, is as described here before for the diol,
   with an oxidizing agent of formula R**OOH wherein R** is hydrogen or a hydrocarbon group that can be substituted and/or interrupted by a heteroatom or heteroatom containing group, or R** is an acyl group of general formula R***-C(=O)- wherein R*** has the same meaning as R**, so as to form a compound of formula R**OH and the epoxide compound to be reacted with water;
- item 27: the method as described in item 26 wherein the oxidizing agent is a peracid of general formula R****-CO₃H where R**** is a hydrocarbon group that can be substituted and/or interrupted by a heteroatom or a heteroatoms-containing group, such as peracetic acid ;
- item 28: the method as described in item 26 wherein the oxidizing agent is hydrogen peroxide ;
- item 29: the method as described in item 28 wherein the olefin is reacted with hydrogen peroxide in the presence of an organic carboxylic acid, such as acetic acid
- item 30: the method as described in any one of items 26 to 29 which comprises:
   - causing a first carboxylic acid of formula 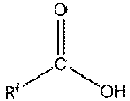 to undergo a Piria ketonization reaction with another carboxylic acid of formula 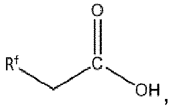
      wherein R^{f}, which may be the same or different in each carboxylic acid, is as described here before,
      wherein the first carboxylic acid and the other carboxylic acid may be consequently identical to or different from each other (they are identical to each other when R^{f} in the formula of the first carboxylic acid is identical to R^{f}-CH₂- in the formula of the other carboxylic acid),
      so as to obtain an internal ketone of formula 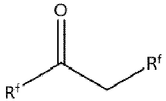 wherein R^{f}, which may be the same or different at each occurrence, is as described here before ;
   - hydrogenating the internal ketone with hydrogen, so as to obtain a carbinol of formula 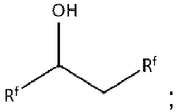 and
   - dehydrating the carbinol, so as to obtain the olefin of formula 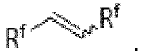
- item 31: use of the diol as described in any one of items 1 to 21 or of the diol mixture as described in item 23 for the manufacture of a surfactant;
- item 32: use of the diol as described in any one of items 1 to 21 for the manufacture of a compound of formula (I) 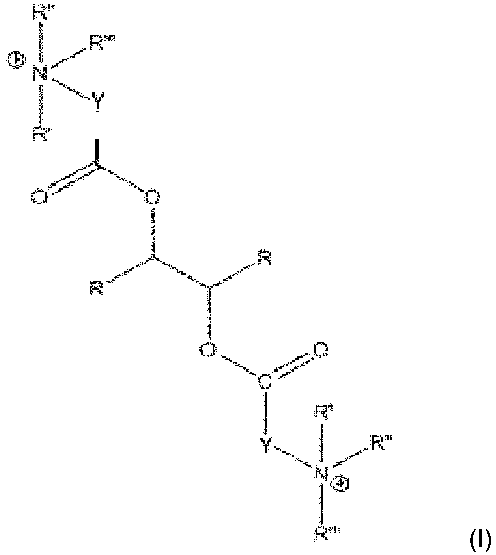 as described hereinbefore (including the meaning of Y, R', R" and R'"), wherein R, which may be the same or different at each occurrence, is equal to R^{f}, as described hereinbefore concerning the diol ;
- item 33: use of the diol as described in any one of items 1 to 21 for the manufacture of a compound other than a compound of formula (I) as described hereinbefore, e.g. for the manufacture of a polymer (possibly an oligomer) such as a polyether or a polyester.

### Esterification (starting from the diol)

The diol can be esterified according to the following reaction scheme: 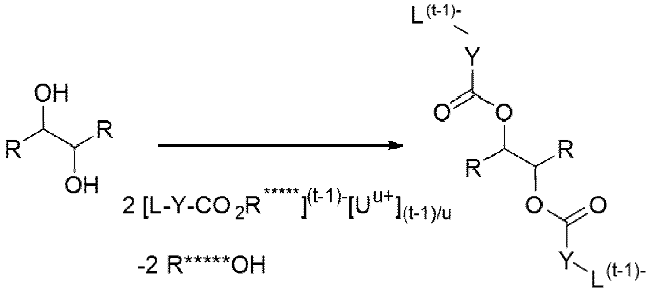 wherein
wherein R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
R***** is hydrogen or a C₁-C₆ alkyl group,
t is an integer which is equal to 1 or which is equal or superior to 2,
U^{u+} is a cation, and
u is an integer fixing the positive charge of the cation.

The esterification is first performed by contacting the diol with an esterification agent which is a carboxylic acid or an ester of a carboxylic acid of general formula:

[L-Y-CO₂R*****]^{(t-1)-}[U^{u+}]_{(t-1)/u}

wherein Y is a divalent hydrocarbon radical containing between 1 and 6 carbon atoms, more precisely a divalent C₁-C₆ aliphatic radical, and wherein L is a leaving group.

Y is preferably an acyclic divalent aliphatic group, more preferably a linear divalent aliphatic group, still more preferably a linear alkanediyl (alkylene) group. Y has preferably from 1 to 6, more preferably from 1 to 4 carbon atom(s), still more preferably 1 or 2 carbon atom(s). The most preferred Y is -CH₂-.

When t is equal to 1, no cation is present. Otherwise said, the esterification is performed by contacting the diol with a carboxylic acid or an ester of a carboxylic acid of formula:

L-Y-CO₂R*****.

In the case the leaving group L already carries a negative charge in the carboxylic acid or ester reactant (this is the case when (t-1) is equal or superior to 1 or when t is equal or superior to 2), a cation noted U^{u+} (with u preferably being 1, 2 or 3, even more preferably 1) must be present in the reactant to ensure the electroneutrality (in this case the cation possesses a u⁺ charge). This cation may e.g. be selected from H⁺, alkaline metal cations, alkaline earth metal cations (e.g. Na⁺, K⁺, Ca ²⁺), Al³⁺ and ammonium, to mention only a few examples.

The nature of the leaving group L is not particularly limited provided next reaction step (i.e. amine condensation, as will be detailed later on) can occur. The leaving group L is preferably chosen from:
- a halogen, such as fluorine, chlorine, bromine or iodine,
- a (hydrocarbyloxysulfonyl)oxy group of formula R^{a}-O-SO₂-O- wherein R^{a} denotes a C₁-C₂₀ hydrocarbyl group, such as CH₃-O-SO₂-O-, and
- an oxysulfonyloxy group of formula -O-SO₂-O-.

An example for a compound with t equal to 1 is the compound CH₃-O-SO₃-CH₂-COOR***** which, for R***** being H, yields the compound CH₃-O-SO₃-CH₂-COOH which can be designated as 2-((methoxysulfonyl)oxy)acetic acid.

As further examples of compounds in which t is equal to 1 and thus no cation is present, one can mention: chloroacetic acid, bromoacetic acid and 2-chloropropionic acid.

An example for t being equal to 2 is sodium carboxymethylsulfate acid in which [L-Y-COOR*****]^{(t-1)-}[U^{u+}]_{(t-1)/u} is [Na⁺][O-SO₂-O-CH₂-COOR*****]⁻ with R***** being H, U being Na and thus [U^{u+}]_{t/u}[L^{t-}] being Na₂SO₄. The esterification can preferably be conducted at a temperature ranging from 50°C to 250°C in the presence of an optional solvent. However the presence of such solvent is not mandatory and the reaction can be also conducted without any added solvent. As example of suitable solvents one can mention: toluene, xylene, hydrocarbons, DMSO, Me-THF, THF or mixtures thereof.

Water that is formed as a by-product during the reaction can be removed from the reaction medium by distillation over the course of the reaction.

A catalyst can also be employed during the reaction and suitable catalysts are Bronsted or Lewis acid catalysts. As preferred examples of catalysts one can mention: H₂SO₄, *para*-toluenesulfonic acid, trifluoromethanesulfonic acid, HCl, or heterogeneous acidic resins such as Amberlite^{®} resins, AlCl₃ etc. At the end of the reaction, the desired diester can be recovered after appropriate work-up and the skilled person is aware of representative techniques so that no further details need to be given here.

As will be seen later on, the diester of formula (IV) 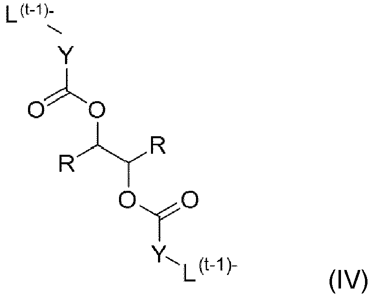 can be easily converted into a compound of formula (I) as previously represented, which exhibits outstanding surfactant properties.

### Direct esterification (starting from the epoxide, including the epoxide ring-opening)

The Applicant has surprisingly found that the epoxide can be directly esterified into the diester when and only when a carboxylic acid is used as the esterification agent [L-Y-CO₂R*****]^{(t-1)-}[U^{u+}]_{(t-1)/u}, that is to say when the esterification agent [L-Y-CO₂R*****]^{(t-1)-}[U^{u+}]_{(t-1)/u} is of the formula [L-Y-CO₂H]^{(t-1)-}[U^{u+}]_{(t-1)/u}, wherein L, Y, t, U^{u+} and u are as described here before in connection with the esterification of the diol.

The epoxide ring-opening and esterification take then place according to the following reaction scheme: 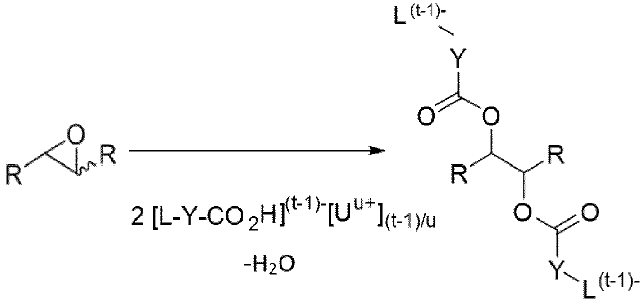 wherein
R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
t is an integer which is equal to 1 or which is equal or superior to 2,
U^{u+} is a cation, and
u is an integer fixing the positive charge of the cation.

The epoxide ring-opening and esterification are performed by reacting the epoxide with a carboxylic acid of general formula:

[L-Y-CO₂H]^{(t-1)-}[U^{u+}]_{(t-1)/u}

wherein L is a leaving group, Y is a divalent C₁-C₆ aliphatic radical, t is an integer which is equal to 1 or which is equal or superior to 2, U^{u+} is a cation, and u is an integer fixing the positive charge of the cation.

All the details which have been provided here before concerning L, Y, t, U^{u+} and U in connection with the esterification of the diol are also valid and can apply here in connection with the direct esterification of the epoxide, so that such details need not to be repeated.

In particular, when t is equal to 1, no cation is present. Otherwise said, the esterification is performed by contacting the epoxide with a carboxylic acid of formula:

L-Y-CO₂H.

### Amine condensation from the diester

The diester compound of formula (IV) can be converted into the ionic compound of formula (I) through the following reaction scheme: 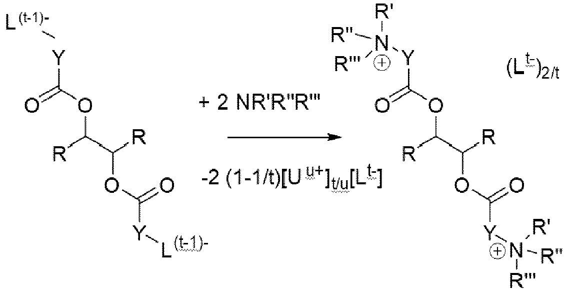 wherein
- R, Y, L, t, U^{u+} and U are as described here before, and
- R', R" and R'" are C₁ to C₄ alkyl groups, preferably methyl or ethyl, most preferably methyl.

The amine condensation reaction is performed by contacting the intermediate diester obtained as described above with an amine of general formula NR'R"R'" where R', R" and R'" are C₁ to C₄ alkyl groups, preferably methyl or ethyl, most preferably methyl.

The reaction can be conducted at a temperature ranging from 15°C to 250°C in the presence of a suitable solvent. As example of a suitable solvent one can mention: THF, Me-THF, methanol, ethanol, isopropanol, DMSO, toluene, xylene or their mixture. Alternatively the reaction can be also conducted in the absence of any added solvent.

During this reaction, there is a nucleophilic attack of the amine that substitues L^{(t-1)-} in the diester, L^{(t-1)-} plays the role of the leaving group. L^{t-} becomes then the counter-anion of the final quaternary ammonium compound. In the case the leaving group already carries a negative charge in the diester reactant (this is the case when (t-1) is equal or superior to 1 or when t is equal or superior to 2) there is also formation of a salt as the by-product of the reaction (with the general chemical formula [U^{u+}]_{t/u}[L^{t-}] as shown in the equation scheme above).

### Esterification and amine condensation / direct quaternization from the diol

Starting from the diol of formula 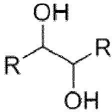 wherein R is as described here before, the Applicant has found that it is possible to obtain in one reaction step a compound which complies with formula (I), i.e. to achieve in one step said esterification and amine condensation, provided another appropriate esterification agent is used. This appropriate esterification agent can be generally defined as an activated form of an amino-acid or an ester thereof of formula

R'R"R'" ⁺N-Y-C(=O)-O⁻

or their hydrohalogenide adducts of formula

R'R"R'" ⁺N-Y-C(=O)-OH X⁻

wherein
- R', R" and R'", which may be the same or different, are hydrogen or a C₁ to C₄ alkyl group,
- Y is a divalent C₁-C₆ aliphatic radical, and
- X denotes a halogen atom, such as chlorine or bromine.

The activated form of the amino-acid or ester thereof or their hydrohalogenide adducts is advantageously an acyl halogenide of formula

R'R"R'" ⁺N-Y-C(=O)-X X⁻

wherein R', R", R'", Y and X are as described before ; in particular, it can be an acyl chloride of formula R'R"R'" ⁺N-Y-C(=O)-Cl Cl⁻.

### Second process for synthesis of compounds represented by formula (I)

### Acyloin condensation

An alternative process for the synthesis of compounds of formula (I) proceeds via an acyloin condensation in accordance with the following scheme: 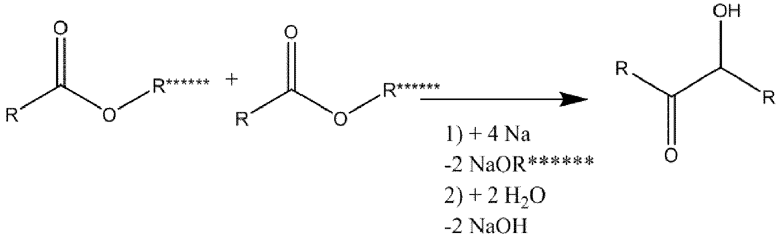 wherein R****** is an alkyl group having from 1 to 6 carbon atoms.

The acyloin condensation is generally performed by reacting an ester (typically a fatty acid methyl ester) with sodium metal as the reducing agent. The reaction be performed in a high boiling point aromatic solvent such as toluene or xylene where the metal can be dispersed at a temperature above its melting point (around 98°C in the case of sodium). The reaction can be conducted at a temperature ranging from 100°C to 200°C. At the end of the reduction, the reaction medium can be carefully quenched with water and the organic phase containing the desired acyloin product can be separated. The final product can be obtained after a proper work-up and the skilled person is aware of representative techniques so that no further details need to be given here.

### Keto-alcohol hydrogenation

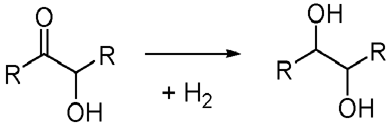

This reaction can be conducted using the conditions described hereinbefore for the first process variant for the manufacture of compounds of formula (I).

The subsequent reaction steps are also as described hereinbefore for the first process variant for the manufacture of compounds of formula (I).

The exemplary processes described before are examples of suitable processes, i.e. there might be other suitable processes to synthesize the compounds in accordance with the present invention. The processes described hereinbefore are thus not limiting as far as the methods of manufacture of the compounds according to the present invention is concerned.

In the compounds of formula (I), the number of carbon atoms of the two groups R is preferably any of the following couples if the internal ketones used as reactant in the exemplary processes described hereinbefore are derived from natural fatty acids having an even number of carbon atoms:
(4,5), (6,7), (8,9), (10,11), (12,13), (14,15), (16,17)
(4,7), (4,9), (4,11), (4,13), (4,15), (4,17)
(5,6), (5,8), (5,10), (5,12), (5,14), (5,16)
(6,9), (6,11), (6,13), (6,15), (6,17)
(7,8), (7,10), (7,12), (7,14), (7,16)
(8,11), (8,13), (8,15), (8,17)
(9,10), (9,12), (9,14), (9,16)
(10,13), (10,15), (10,17)
(11,12), (11,14), (11,16)
(12,15), (12,17)
(13,14), (13,16)
(14,17)
(15,16).

In particular, if the internal ketones used as reactant in the exemplary processes described hereinbefore are obtained from one or more carboxylic acids having an even number of carbon atoms ranging from 12 to 18, the number of carbon atoms of the two groups R in compounds of formula (I) is one or more of the following couples:
(10,11), (12,13), (14,15), (16,17)
(10,13), (10,15), (10,17)
(11,12), (11,14), (11,16)
(12,15), (12,17)
(13,14), (13,16)
(14,17)
(15,16)

If the internal ketone is obtained from fatty acids comprising an odd numbers of carbon atoms, other couples are possible and will be obtained.

Compounds of formula (I) possess a particularly interesting and advantageous property profile of surfactant properties on one hand and biodegradability properties on the other hand. As biodegradability is becoming more and more an important aspect for surfactant products, compounds of formula (I) constitute a preferred embodiment of the present invention.

The compounds of the present invention can be used as surfactants. Surfactants are compounds that lower the surface tension (or interfacial tension) between two liquids, a liquid and a gas or between a liquid and a solid. Surfactants may act as detergents, wetting agents, emulsifiers, foaming agents, and dispersants.

Surfactants are usually organic compounds that are amphiphilic, meaning they contain both hydrophobic groups (their *tails*) and hydrophilic groups (their *heads*). Therefore, a surfactant contains both a water-insoluble (or oil-soluble) component and a water-soluble component. Surfactants will diffuse in water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase.

The adsorption of a cationic surfactant on negatively charged surfaces is an important property for such surfactants. This property is usually linked to the minimum concentration of surfactant needed to produce aggregation of a negatively charged cellulose nanocrystal (CNC, which is often used as reference material)) suspension in aqueous media. Consecutive variation of size can be monitored and followed by dynamic light scattering (DLS).

Following the protocol described in E.K. Oikonomou et al., J. Phys. Chem. B, 2017, 121 (10), 2299-307 the adsorption properties of the quaternary ammonium compounds can be investigated by monitoring the ratio X=[surfactant]/[CNC] or the mass fraction M=[surfactant]/([surfactant + [CNC]), at fixed [surfactant] + [CNC] = 0.01 wt% in aqueous solution, required to induce the agglomeration of the cellulose nanocrystals.

The biodegradability of the compounds of the present invention can be determined in accordance with procedures described in the prior art and known to the skilled person. Details about one such method, OECD standard 301, are given in the experimental section hereinafter.

Should the disclosure of any patents, patent applications, and publications which are incorporated herein by reference conflict with the description of the present application to the extent that it may render a term unclear, the present description shall take precedence.

### Working examples

### Example 1 - Synthesis of a quaternary ammonium compound corresponding to formula (I) starting from C₃₁ 16-hentriacontanone

C₃₁ internal olefin was obtained from palmitic acid according to the protocol described in US patent 10035746, example 4.

### Epoxidation of internal olefin to fatty epoxide

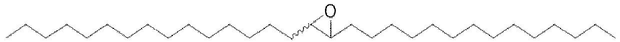

The reaction was conducted under an inert argon atmosphere.

In a 1L double-jacketed reactor equipped with a mechanical stirrer (propeller with four inclined plows), a condenser, an addition funnel and a temperature probe were added 61.9 g of C₃₁ alkene (0.142 mol), followed by 16.3 mL (17.1 g, 0.285 mol) of acetic acid and 13.6 g (22 wt%) of Amberlite^{®} IR 120H resin. The mixture was heated to 65 °C to melt the fatty alkene. The agitation was started and then 21.8 mL (24.2 g, 0.214 mol) of an aqueous solution of H₂O₂ (conc. 30%) was slowly added to the mixture using the addition funnel at a rate avoiding a significant temperature increase. This required about one hour. The temperature was then increased to 75°C and the reaction mixture was allowed to stir overnight (after 15 min, NMR analysis showed that the conversion level was already around 60% with 99% selectivity). Then additional 10.2 mL (11.3 g, 0.1 mol) of an aqueous solution of H₂O₂ (30%) was added slowly and after 4 hours following the second addition of H₂O₂ NMR analysis showed that the conversion level was around 88% (98% selectivity). Another addition of 8.14 mL of acetic acid (8.55 g, 0.142 mol) followed by 11.6 mL of 30% H₂O₂ (12.91 g, 0.114 mol) was finally performed in order to increase the conversion level.

The mixture was allowed to stir a second night at 75°C.

Finally NMR analysis showed a conversion level of 93% (95% selectivity).

The mixture was allowed to cool down to room temperature and then 300 mL of chloroform were added. The mixture was transferred to a separating funnel and the organic phase was washed three times with 300 mL of water and then the aqueous phase was extracted twice with 100 mL of chloroform. The Amberlite^{®} solid catalyst stayed in the aqueous phase and was removed during the first separation with the aqueous phase. The organic phases were collected, dried over MgSO₄, filtered and evaporated to give 65.3 g of a white solid with a purity of 91% w/w (epoxide + di-alcohol).

The yield taking into account the purity was 92%.

¹H NMR (CDCl₃, 400 MHz) δ (ppm): 2.91-2.85 (m, 2H, diastereoisomer 1), 2.65-2.6 (m, 2H, diastereoisomer 2), 1.53-1.00 (m, 54H), 0.86 (t, J = 6.8 Hz, 6H).

¹³C NMR (CDCl₃, 101 MHz) δ (ppm): 58.97, 57.28, 32.18, 31.96, 29.72, 29.6, 29.4, 27.86, 26.95, 26.63, 26.09, 22.72, 14.15 (terminal CH₃).

Hydrolysis of fatty epoxide to afford fatty diol

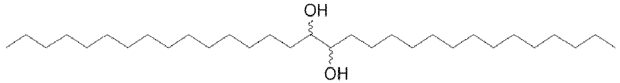

The reaction was conducted under an inert argon atmosphere.

In a 1L double-jacketed reactor equipped with a mechanical stirrer (propeller with four inclined plows), a condenser and a temperature probe were added 82.9 g of C₃₁ epoxide (purity: 94.5 wt%, 0.174 mol) followed by 480 mL of methyl-THF.

The mixture was allowed to stir at room temperature and 73 mL of a 3 M aqueous solution of H₂SO₄ was then added. The reaction medium was then stirred at 80°C during 90 minutes. NMR analysis showed that the reaction was completed. The biphasic mixture was allowed to cool down to room temperature and the organic phase was separated. The solvent was then removed under vacuum and the residue was suspended in 200 mL of diethyl ether. The suspension was filtered and the resulting solid was washed 3 times with 50 mL of diethyl ether. The white solid was finally washed 2 times with 50 mL of methanol and was dried under vacuum to remove traces of solvent.

At the end 75.53 g of product was obtained as a white powder with a purity of 95.7 % w/w corresponding to a yield of 89 %.

¹H NMR (CDCl₃, 400 MHz) δ (ppm): 3.61-3.55 (m, 2H, diastereoisomer 1), 3.43-3.25 (m, 2H, diastereoisomer 2), 1.88 (brd, *J* = 2.4 Hz, OH, diastereoisomer 2), 1.72 (brd, *J* = 3.2 Hz, OH, diastereoisomer 1), 1.53-1.10 (m, 54H), 0.86 (t, *J* = 6.8 Hz , 6H).

¹³C NMR (CDCl₃, 101 MHz) δ (ppm): 74.71, 74.57, 33.66, 31.96, 31.23, 29.71, 29.39, 26.04, 25.68, 22.72, 14.15 (terminal CH₃)

### Esterification and quaternization of fatty diol with trimethylglycine to afford compound of formula (I)

All the reactions were conducted in carefully dried vessels and under an inert argon atmosphere.

Fresh commercial anhydrous CHCl₃ (amylene stabilized) and anhydrous toluene were used as such.

Betaine hydrochloride (19.66 g, 128.4 mmoles) was washed ten times with 20 mL of anhydrous THF followed by drying under vacuum to remove traces of solvent prior to use.

In a 100 mL four-neck round-bottom flask equipped with a magnetic stirrer, a heater, a condenser, a temperature probe and a curved distillation column connected to two traps of NaOH were quickly added:
19.66 g of dried betaine hydrochloride (128.4 mmoles) and 28 mL of SOCl₂ (45.86 g, 0.386 mol).

The heterogeneous mixture was stirred and the temperature was then slowly increased to 70°C. It was observed that when the temperature reached 68°C, gas was released (SO₂ and HCI) and the mixture turned homogeneous yellow.

The mixture was then allowed to stir at 70°C during two hours and hot anhydrous toluene (25 mL, 80°C) was added into the vessel. The mixture was stirred and then decanted at 0°C (white-yellow precipitate formation) and the upper phase of toluene was removed through a cannula. The operation of toluene washing was repeated seven times in order to remove all SOCl₂ excess. NMR analysis showed complete conversion of glycine betaine hydrochloride but also formation of NMe₃·HCl adduct (NMe₃·HCl content in the solid: 12.3 mol%).

20 mL of dry CHCl₃ was then added to the solid betainyl chloride.

A solution of 26.19 g (56 mmol) of fatty diol in 90 mL of anhydrous CHCl₃ was prepared at 55°C and was added dropwise under stirring to the reaction vessel at room temperature (exothermicity and emission of HCl was observed). The mixture was then allowed to stir at 55°C overnight. Over the course of the reaction, the mixture turned homogeneously orange. NMR analysis showed that the conversion level was around 100%.

The mixture was then allowed to cool down to room temperature and the solvent was evaporated under vacuum.

The residue was solubilized in methanol at 0°C and the formed precipitate was filtered out. The obtained filtrate was then evaporated to give 39.7 g of crude product.

This product was then deposited on a sinter filter and washed with cyclohexane to remove some remaining organic impurities. The resulting washed solid was dried under vacuum to afford 22 g of crude material. A final purification with a mixture of CH₂Cl₂/cyclohexane 50:50 was carried out; the solid was solubilized again in this solvent mixture at 50°C and was allowed to cool down to room temperature. The formed precipitate was filtered out and after evaporation of the filtrate 19 g of a beige wax was obtained with the following composition:
95 wt% of glycine betaine diester
1.5 wt% of methyl betainate
2 wt% of trimethylamine hydrochloride
1.5 wt% of glycine betaine hydrochloride.

The purified yield was 44%.

¹H NMR (MeOD-d4, 400 MHz) δ (ppm): 5.3-5.2 (m, 2H), 4.68 (d, J = 16.8 Hz, 2H), 4.50 (d, J = 16.8 Hz, 2H), 4.53 (s, 1H), 4.48 (s, 1H), 3.37 (s, 18H), 1.75-1.55 (m, 4H), 1.39-1.10 (m, 50 H), 0.9 (t, J = 6.8 Hz, 6H).

¹³C NMR (MeOD-d4, 101 MHz) δ (ppm): 164.58, 75.76, 62.43, 53.10, 31.68, 30.05, 29.41, 29.38, 29.33, 29.28, 29.15, 29.09, 28.96, 24.71, 22.34, 13.05 (terminal CH₃).

### Example 2 - Evaluation of adsorption properties on Nanocellulose crystals

Adsorption of cationic surfactant on negatively charged surface is an important property for various applications. This property is linked to the minimal concentration of cationic surfactant needed to produce aggregation of negatively charged cellulose nano crystal (CNC) in suspension in aqueous media. Comparison of the aggregate size can be monitored by dynamic light scattering (DLS).

Following the protocol described in literature (Ref.: E. K. Oikonomou, et al., J. Phys. Chem. B, 2017, 121 (10), pp 2299-2307), adsorption properties of quaternary ammonium were investigated by monitoring the ratio X=[surfactant]/[CNC] or the mass fraction M = [surfactant]/([surfactant]+[CNC]), at fixed [surfactant]+[CNC]= 0.01wt% in aqueous solution, required to induce the agglomeration of the cellulose nano crystal.

The range of CNC aggregation correspond to the range of ratio X (or M) triggering an aggregation of CNC, i.e. the range where the aggregate size measured by DLS is higher than a pure aqueous solution of CNC or an aqueous solution of surfactant at 0.01wt%.

Ranges of X and M of aggregation of CNC are summarized in Table 1. The lower range of aggregation X or M, the better the adsorption properties on negatively charged surface

**Table 1**

| | Range of CNC aggregation (Ratio) X=[surfactant]/[CNC] Xₘᵢₙ - Xₘₐₓ | Range of CNC aggregation (Mass fraction) Mₘᵢₙ - Mₘₐₓ |
|---|---|---|
| Compound of Example 1 | 0.1-1.82 | 0.09-0.65 |
| Fentacare^{®} TEP ¹ | 1-33 | 0.50-0.97 |

| | | |
|---|---|---|
| ¹ Fentacare^{®} TEP was used as a comparison. Fentacare^{®} TEP is a commercial surfactant representing the benchmark. | | |

The data show that the surfactant properties of the compound in accordance with the present invention is superior compared to the commercial surfactant Fentacare^{®} TEP.

### Example 3 - Determination of biodegradability

Biodegradability of the test substances has been measured according to the 301 F OECD protocol.

A measured volume of inoculated mineral medium, containing a known concentration of the test substance in order to reach about 50 to 100 mg ThOD/I (Theoretical Oxygen Demand) as the nominal sole source of organic carbon, was stirred in a closed flask (Oxitop^{™}respirometric flask) at a constant temperature (20 ± 2°C) for up to 28 days. Oxitop^{™} respirometric bottles were used in this test in order to access the biodegradability of the test samples: sealed culture BOD flasks were used at a temperature of 20±2 C during 28 days.

Evolved carbon dioxide was absorbed by pellets of Natrium or Potassium hydroxide present in the head space of the bottle. The amount of oxygen taken up by the microbial population (= oxygen consumption expressed in mg/l) during biodegradation process (biological oxidation of the test substance) decreased the pressure of the head space (Δ P measured by the pressure switch) and was mathematically converted in mg O₂ consumed /litre. Inoculum corresponded to a municipal activated sludge washed in mineral medium (ZW media) in order to decrease the DOC (Dissolved Oxygen Carbon) content. Control solutions containing the reference substance sodium acetate and also toxicity control (test substance + reference substance) were used for validation purposes. Reference substance, sodium acetate, has been tested in one bottle (at a nominal concentration of 129 mg/l corresponding to 100 mg ThOD/I) in order to check the viability of the inoculum. Toxicity control corresponds to the mixture of the substance reference and the test substance; it will check if the test substance is toxic towards the inoculum (if so, the test has to be redone at a lower test substance concentration, if feasible regarding the sensitivity of the method).

As the substances of the present invention are for a majority of them not very soluble in water (if some are soluble in water, their metabolite after hydrolysis containing the alkyl chain has often very low solubility in water), we used a specific protocol named the "emulsion protocol". This protocol enabled us to increase the bioavailability of the poorly water soluble substance in the aqueous phase where we had the inoculum.

Emulsion protocol consisted of adding the test substance in the bottle through a stock solution made in an emulsion.

Emulsion was a 50/50 v/v mixture of a stock solution of the test substance dissolved in a non-biodegradable surfactant (Synperonic^{®} PE 105 at 1 g/l) and then mixed with a mineral silicone oil AR 20 (Sigma).

The first dissolution of the test substance in the non-biodegradable surfactant solution often required magnetic stirrer agitation followed by ultrasonication.

Once the dissolution was made, we mixed the aqueous solution with a mineral silicone oil at a 50/50 volume/volume ratio. This emulsion was maintained by magnetic stirrer agitation and was sampled for an addition in the corresponding bottle in order to reach the required test substance concentration.

Two emulsion controls were run in parallel during the test in order to remove their value from the emulsion bottle containing the test substance added through the emulsion stock solution.

The results of the biodegradability test is provided in Table 2 here below:

**Table 2**

| | Biodegradability after 28 days |
|---|---|
| Compound of Example 1 | 92 % (OECD 301F) |

The result shows that the compound of example 1 has outstanding biodegradability. This beneficial effect is achieved without detrimentally affecting the surfactant properties of the compounds.

Overall, the compounds of the present invention show a good combination of surfactant properties combined with a reasonable to good biodegradabilty - a combination which is in many cases not achieved by commercial surfactants.

Since the compounds of the present invention are also easily available starting from internal ketones which are easily accessible from fatty acids or fatty acid derivatives, the compounds of the present invention also provide economical benefits over the prior art compounds.

## Claims

1. A diol of formula (III) 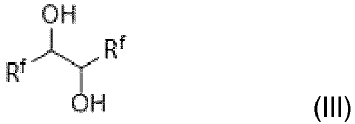
wherein R^{f}, which may be the same or different at each occurrence, represents a C₁₀-C₂₇ aliphatic group,
with the exception of docosane-11,12-diol and heptacosane-13,14-diol.

2. The diol according to claim 1 with the further exception of branched tricosane-11,12-diol, branched tetracosane-11,12-diol, branched tetracosane-12,13-diol, branched pentacosane-12,13-diol, branched hexacosane-12,13-diol, branched hexacosane-13,14-diol, branched heptacosane-12,13-diol, branched octacosane-13,14-diol, branched octacosane-14,15-diol, linear or branched nonacosane-14,15-diol and branched triacontane-15,16-diol.

3. The diol according to claim 1 or 2 wherein R^{f} is linear.

4. The diol according to claim 3 wherein R^{f} is a linear alkyl group.

5. The diol according to any one of claims 1 to 4 wherein R^{f} comprises from 10 to 20 carbon atoms.

6. The diol according to claim 1 wherein R^{f} is a linear alkyl group having from 14 to 17 carbon atoms.

7. The diol according to any one of claim 1 to 6 which is a C₃₃ diol.

8. A pack of at least one kilogram comprising the diol according to any one of the preceding claims.

9. A diol mixture comprising a first diol **characterized by** R^{f} groups having respectively n₃ and (n₃-1) carbon atoms, a second diol **characterized by** R^{f} groups having respectively n₄ and (n₄-1) carbon atoms, a third diol **characterized by** R^{f} groups having respectively n₃ and (n₄-1) carbon atoms and a fourth diol **characterized by** R^{f} groups having respectively n₄ and (n₃-1) carbon atoms, wherein n₃ ≤ n₄ + 2 and wherein the first diol, the second diol, the third diol and the fourth diol are as claimed in any one of claims 1 to 6.

10. A method for obtaining the diol according to any one of claims 1 to 7 which comprises reacting an epoxide compound of formula 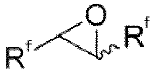
wherein R^{f}, which may be the same or different at each occurrence, is as defined for the diol,
with water.

11. The method according to claim 10 wherein the reaction is conducted in the presence of a Bronsted or Lewis catalyst.

12. The method according to claim 10 or 11 which comprises reacting an olefin of formula 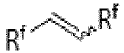
wherein R^{f}, which may be the same or different at each occurrence, is as defined for the diol,
with an oxidizing agent of formula R**OOH wherein R** is either hydrogen or a hydrocarbon group or an acyl group of general formula R***-C(=O)- wherein R*** is either hydrogen or a hydrocarbon group, so as to form a compound of formula R**OH and the epoxide compound to be reacted with water;

13. The method according to claim 12 which comprises:
- causing a first carboxylic acid of formula 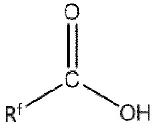 to undergo a Piria ketonization reaction with another carboxylic acid of formula 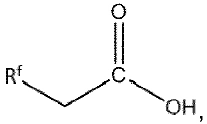
wherein R^{f}, which may be the same or different in each carboxylic acid, is as defined for the diol,
wherein the first carboxylic acid and the other carboxylic acid may be identical to or different from each other,
so as to obtain an internal ketone of formula 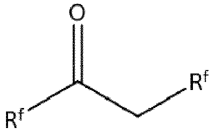
- hydrogenating the internal ketone with hydrogen, so as to obtain a carbinol of formula 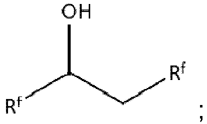 and
- dehydrating the carbinol, so as to obtain the olefin of formula

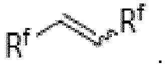

14. Use of the diol according to any one of claims 1 to 7 or of the diol mixture according to claim 9 for the manufacture of a surfactant.

15. Use according to claim 14 wherein the surfactant is a compound of formula (I) 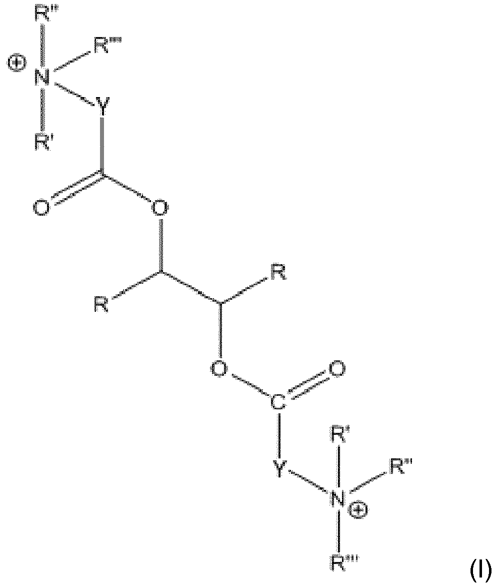 wherein, in formula (I):
R, which may be the same or different at each occurrence, is equal to R^{f}, with R^{f} being as defined for the diol,
Y, which may be the same or different at each occurrence, is a divalent C₁-C₆ aliphatic radical, and
R', R" and R'", which may be the same or different at each occurrence, are, independently from each other, hydrogen or a C₁ to C₄ alkyl group.
